# EUROPÄISCHE PATENTSCHRIFT

(11) **EP 2 978 394 B1**
(45) Veröffentlichungstag und Bekanntmachung des Hinweises auf die Patenterteilung: **12.06.2019**
(21) Anmeldenummer: 14700509.4
(22) Anmeldetag: 15.01.2014
(51) Int. Cl.: A61F 9/02, G02C 9/00

(54) **SICHTSCHEIBE FÜR EINE BRILLE ODER HELMVISIER**
VIEW PANEL FOR GOGGLES OR HELMET VISOR
VERRE DE VUE POUR LUNETTES OU VISIÈRE DE CASQUE

(30) Priorität: 26.03.2013 AT 502072013
(43) Veröffentlichungstag der Anmeldung: 03.02.2016
(73) Patentinhaber: Rauter, Christoph, 1070 Wien (AT)
(72) Erfinder: Rauter, Christoph, 1070 Wien (AT)
(74) Vertreter: Puchberger & Partner Patentanwälte
(86) Internationale Anmeldenummer: PCT/EP2014/050718
(87) Internationale Veröffentlichungsnummer: WO 2014/154370

(56) Entgegenhaltungen:
- EP-A2- 0 338 524
- WO-A1-99/44555
- US-A- 2 263 116
- US-A- 3 533 686
- US-A- 5 018 223
- US-A- 5 371 555
- US-A- 6 009 564
- US-A1- 2008 170 202

## Beschreibung

Die Erfindung betrifft eine Sichtscheibe für eine Brille, vorzugsweise eine Sportbrille, Skibrille oder Schutzbrille, oder für ein Helmvisier, gemäß dem Oberbegriff des Anspruchs 1, sowie eine Brille mit einer derartigen Sichtscheibe.

Eine gattungsgemäße Sichtscheibe ist aus der WO99/44555 A bekannt. Die Sichtscheibe umfasst eine Innenscheibe und eine Außenscheibe wobei die Außenscheibe in einem Abstand von der Innenscheibe angeordnet ist und mit dieser über ein randseitiges Dichtungsmittel verbunden ist. Die Innenscheibe bezeichnet dabei die bei Benutzung den Augen näher liegende Scheibe, und die Außenscheibe die bei Benutzung den Augen ferner liegende Scheibe. Derartige Sichtscheiben werden für Skibrillen und Motorradhelmvisiere benutzt. Sie ermöglichen es, für die inneren und die äußeren Scheiben unterschiedliche Materialien zu verwenden, beispielsweise für die Außenscheibe besonders kratzfestes Material und für die Innenscheibe Material, das sich gut mit einer Anti-Beschlag-Beschichtung versehen lässt. Weiterhin wird durch den eingeschlossenen Luftpolster im Zwischenraum zwischen der Innenscheibe und der Außenscheibe ebenfalls einem Beschlagen der Scheibe entgegengewirkt. Diese bekannten Sichtscheiben erschweren jedoch fehlsichtigen Personen das Tragen optischer Brillen zur Korrektur der Fehlsichtigkeit. In der Regel ist der vom Rahmen der Brille umgrenzte Hohlraum nicht groß genug, um die benötigte optische Brille unter der Skibrille zu tragen.

Zu diesem Zweck ist es aus der DE 8519305U1 bekannt, im Rahmen der Skibrille Klemmvorrichtungen anzuordnen, in die optische Linsen mittels seitlich angeordneter Abstützelemente (sogenannte Innenclips) eingeklemmt werden können. Die derzeit dafür verwendeten Innenclips für optische Linsen werden hinter der Doppelscheibe in den Rahmen der Skibrille eingesetzt.

Derartige in den Hohlraum zwischen dem Gesicht des Benutzers und der Sichtscheibe der Skibrille eingeklemmte optische Linsen sind jedoch im Vergleich zu den optischen Linsen herkömmlicher Brillen in ihrem Durchmesser deutlich limitiert, wodurch das periphere Sehen stark eingeschränkt wird. Zudem kommt es häufig zum Beschlagen der optischen Linsen. Auch das Streifen der Wimpern des Benutzers an den hervorstehenden optischen Linsen stellt immer wieder ein Problem beim Tragen derartiger Innenclips dar.

Gattungsgemäße Sichtscheiben, welche über eine Außenscheibe, eine Innenscheibe und eine dazwischen angeordnete optische Linse verfügen, sind für verschiedenste Einsatzzwecke aus den Druckschriften US 2,263,116 A, US 5,371,555 A, US 3,533,686 A sowie EP 0 338 524 A2 bekannt. Eine mehrschichtige Skibrille ist aus der US 5,018,223 A bekannt.

Der Erfindung liegt demnach die Aufgabe zugrunde, eine Sichtscheibe der eingangs genannten Art so weiterzubilden, dass sie einerseits vorteilhafte Gebrauchseigenschaften sicherstellt und andererseits die optische Korrektur von Fehlsichtigkeiten erlaubt.

Dies wird erfindungsgemäß bei einer Sichtscheibe gemäß Oberbegriff des Anspruchs 1 durch die kennzeichnenden Merkmale des Anspruchs 1 erreicht. Bei der erfindungsgemäßen Sichtscheibe ist in der Innenscheibe zumindest eine optische Linse zur Korrektur von Fehlsichtigkeiten integriert.

Im Unterschied zu den derzeit verwendeten optischen Innenclips wird der Durchgang des Lichtes in Summe anstatt von 6 lichtbrechenden Oberflächen nur noch von 4 lichtbrechenden Oberflächen beeinflusst. Zudem kann die optische Linse entspiegelt werden und so die Lichttransmission erhöht bzw. störende Lichtreflexe reduziert. Dies führt zu einem stressfreieren Sehen und zu Energiereserven in der Sportausübung. Der Abstand der optischen Linse zum Auge des Benutzers kann weitgehend dem Abstand von "normalen" optischen Brillengläsern angepasst werden. Damit werden Umstellungsschwierigkeiten beim Wechsel zwischen den Brillen deutlich reduziert.

Das Gesichtsfeld wird im Vergleich zu einem Innenclip deutlich vergrößert und die Wahrnehmung verbessert. Das übliche Brillenskotom eines Innenclips wird verhindert, und das Gesichtsfeld des Benutzers wird ohne den zusätzlichen Rahmen nicht negativ beeinträchtigt. Auch ist ein Verrutschen der optischen Linse nicht möglich.

Durch den größeren Abstand der Linsen zur Augenoberfläche wird auch ein Streifen der Wimpern an der Oberfläche der Linsen und damit ein permanentes Verschmutzen verhindert. Da die optische Linse nicht in der Außenscheibe eingebracht ist, verhindert man eine rasche Zerstörung aufgrund von Umwelteinflüssen. Darüber hinaus wird durch die Integration der Linse in die Innenscheibe Schmutz, Wasser, Staub oder Schnee auf der Außenscheibe nicht optisch verzerrt abgebildet. Auch wird die Linse vor Verunreinigung und mechanischen Beeinträchtigungen durch Steinschlag, Äste, oder sonstige Fremdkörper geschützt.

Die Doppelscheibenkonstruktion bietet den Vorteil einer hohen Verwindungssteifigkeit. Durch die Integration der optischen Linse in die Doppelscheibe können optische Linsen mit großem Durchmesser in die Scheibe integriert werden. Damit ermöglicht die Erfindung eine Ausweitung des Gesichtsfeldes sowie eine Verbesserung des peripheren Sehens und trägt zu einer Erhöhung der Sicherheit bei.

Dabei umfasst der Ausdruck optisches Linse eine durchsichtige Scheibe zur Korrektur von Fehlsichtigkeiten aus beliebigem Material, beispielsweise aus Kunststoff, Glas, CR-39, Polycarbonat, oder Kombinationen dieser Materialien. Besonders geeignet sind Scheiben aus Kunststoff der Indizes 1,59 bis 1,67, Polycarbonat, oder Trivex.

Zur Aufnahme der optischen Linse kann die Innenscheibe zumindest eine Ausnehmung aufweisen, die den Einbau der Linse erlaubt. Die Linse kann in die Ausnehmung geklebt sein. Die Ausnehmung kann vorzugsweise gestanzt oder gefräst sein. In der Ausnehmung können erste Befestigungsmittel zur Aufnahme der Linse vorgesehen sein. Die Linse kann korrespondierende zweite Befestigungsmittel aufweisen.

Erfindungsgemäß kann die optische Linse gerillt und durch ein Nut und Feder System in die innere Scheibe integriert sein. Insbesondere kann vorgesehen sein, dass die Linse größer ist als die zugehörige Ausnehmung und eine umlaufende Nut zum Einschieben von zumindest Teilen des umlaufenden Randes der Ausnehmung aufweist, sodass die Linse beim Zusammenbau in die Ausnehmung gesteckt werden kann.

Erfindungsgemäß ist weiters vorgesehen dass die Innenscheibe und die Außenscheibe lösbar miteinander verbunden sein können. Durch das Nut und Feder System und die lösbare Verbindung der Innenscheibe mit der Außenscheibe wird ein einfacher nachträglicher Tausch der optischen Linse ermöglicht.

Die umlaufende Nut kann als Nutfräsung mit einer Breite von 0,40mm bis 1,20mm, vorzugsweise 0,80mm und einer Tiefe von 0,20mm bis 1,00 mm, vorzugsweise 0,60mm ausgeführt sein. Die optische Linse kann zusätzlich mittels UV-Klebers gesichert sein.

Die Nut kann im Wesentlichen parallel zum Verlauf jenes umlaufenden Randes der optischen Linse ausgeführt sein, der im Zwischenraum zwischen Innenscheibe und Außenscheibe liegt. Insbesondere kann die Nut vorzugsweise parallel zur Vorderfläche der Linse verlaufen. Die optische Linse sollte zur Aufrechterhaltung eines Luftpolsters die Außenscheibe nicht berühren, um Beschlagen durch Temperaturunterschiede zu verhindern. Zu diesem Zweck kann vorgesehen sein, dass die Linse derart in der Innenscheibe integriert ist, dass der Vorsprung der Linse in den Zwischenraum zwischen Innenscheibe und Außenscheibe maximal der Hälfte der Dicke des Zwischenraumes entspricht. Die Dicke des Zwischenraums kann etwa 3mm bis 5mm betragen.

Die Außenscheibe und die Innenscheibe können gekrümmt, vorzugsweise kugelförmig gekrümmt sein. Es ist vorgesehen, dass die Frontkurve der optischen Linse im Zwischenraum zwischen der Innenscheibe und der Außenscheibe im Wesentlichen parallel zur Frontkurve der Außenscheibe verläuft, um Abbildungsfehler zu vermeiden. Dabei können die Außenscheiben und Innenscheiben derart geformt sein, dass ein größtmögliches Maß an Verwindungssteifigkeit der Doppelscheibe erhalten bleibt.

Die Außenscheibe kann Cellulosepropionat, Celluloseacetat oder Polycarbonat umfassen. Die Innenscheibe kann Cellulosepropionat oder Celluloseacetat umfassen. Die Scheiben können aber auch aus anderen in der Augenoptik gebräuchlichen Materialien gefertigt sein. Das Dichtungsmittel kann als ein längs der Randbereiche der Scheiben umlaufendes, elastisches Abstandhalte-Dichtungselement ausgeführt sein, welches mit der Außenscheibe und der Innenscheibe verbunden, vorzugsweise verklebt ist. Das Abstandhalte-Dichtungselement kann insbesondere als Schaumstoff-Dichtring ausgeführt sein. Der Schaumstoff-Dichtring kann eine Breite von 3mm bis 5mm und eine Dicke von 3mm bis 5mm aufweisen und mittels eines Doppelklebebandes mit den Scheiben verklebt sein.

Die Außenscheibe, die Innenscheibe und/oder die optische Linse können eine beschlaghemmende Beschichtung umfassen. Insbesondere kann die Innenscheibe und/oder die Linse beidseitig mit einer beschlaghemmenden Beschichtung versehen sein. Die Außenscheibe und/oder die Innenscheibe kann darüber hinaus mit einer lichtreflektierenden bzw. Infrarotstrahlung reflektierenden Beschichtung versehen sein. Die Außenscheibe kann eine Wandstärke von 0,80mm bis 1,20mm aufweisen. Die Innenscheibe kann eine Wandstärke von 0,60mm bis 0,80mm aufweisen.

Vorzugsweise kann die Sichtscheibe derart ausgeführt sein, dass in der Innenscheibe zwei Ausnehmungen zur Integration optischer Linsen vorgesehen sind. In Abhängigkeit vom Augenabstand (Pupillardistanz), der Stärke der Fehlsichtigkeit sowie der Sportart bzw. des Einsatzbereiches ist erfindungsgemäß vorgesehen dass die Ausnehmungen in ihrer Größe und Position variiert werden können. Der minimale Abstand der Ausnehmungen bzw. der darin befindlichen optischen Linsen kann 11mm bis 15mm, vorzugsweise 13mm betragen, um eine zentrale Blickeinschränkung zu verhindern. Die optischen Linsen können zur Korrektur von Kurzsichtigkeit, zur Korrektur von Fernsichtigkeit, als Bifokalgläser, oder als Gleitsichtgläser ausgeführt sein.

Das Zusammenführen der Außenscheibe und Innenscheibe zur Doppelscheibe kann mittels Verkleben nach dem Einbau der optischen Linsen in die Innenscheibe erfolgen. Hierzu ist die Verwendung einer Dichtung in Form eines Schaumstoff-Dichtringes, der mittels Doppelklebefunktion bereits an der Außenscheibe geklebt ist, vorteilhaft. Nach Fertigung der Innenscheibe mit den optischen Linsen, kann diese, nach Entfernung des Schutzpapiers vom Schaumstoff-Dichtring, an markierten Stellen aufgebracht und beide Scheiben über diesen Schaumstoff-Dichtring fest miteinander verklebt werden.

Die optischen Linsen werden vorzugsweise mit einer Nutfräsung so vorbereitet, dass die Innenscheibe in die optischen Linsen gesteckt werden kann. Zur besseren Abdichtung gegenüber dem Hohlraum zwischen den Scheiben der Doppelscheibe können die optischen Linsen zusätzlich mittels UV-Kleber gesichert werden.

Der Einbau der optischen Linsen führt zu einer homogen geschlossenen Innenscheibe und erzielt im verklebten Zustand die positiven Effekte einer Doppelscheibe, nämlich eine deutlich reduzierte Wahrscheinlichkeit des Beschlagens und eine hohe Verwindungssteifigkeit. Durch die in die Doppelscheibe integrierten optischen Linsen und durch die großflächige Auflagefläche im Gesicht ist eine stabile optische Abbildung ohne Verrutschen sichergestellt.

Weitere erfindungsgemäße Merkmale ergeben sich aus der Beschreibung der Ausführungsbeispiele, den Figuren, und den Ansprüchen.

Die Erfindung wird nun unter Bezugnahme auf Ausführungsbeispiele, die in den Zeichnungen schematisch dargestellt sind, erläutert.

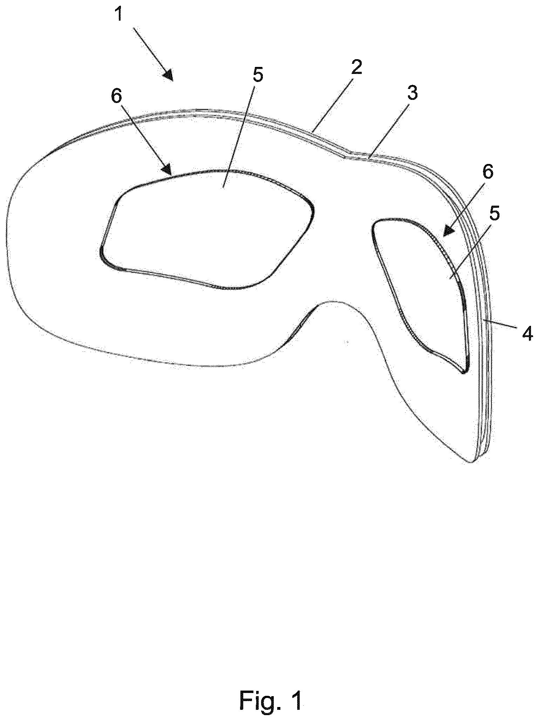

Fig. 1 zeigt ein erstes Ausführungsbeispiel einer erfindungsgemäßen Sichtscheibe 1 aus Sicht des Benutzers. Die Sichtscheibe 1 umfasst eine Außenscheibe 2 und eine Innenscheibe 3. Zwischen den Scheiben 2, 3 ist ein Schaumstoff-Dichtring 4 angeordnet. Dieser garantiert die Stabilität der Doppelscheibe und dichtet den Luftpolster zwischen den Scheiben ab. Die Innenscheibe 3 verfügt über zwei Ausnehmungen 6, in die optische Linsen 5 eingesetzt sind. In Abhängigkeit vom Augenabstand (Pupillendistanz) kann die Ausnehmung für die optischen Linsen in unterschiedlichen Scheibenlängen erfolgen. Der Schaumstoff-Dichtring 4 wird ringförmig entlang der Außenkante der Scheiben 2, 3 angebracht und dient auch als Abstand halter.

Durch die heute verfügbaren modernen Brillenglastechnologien kann die Frontkurve der optischen Linse 5 (jene Oberfläche, die der Außenscheibe 2 zugewandt ist) an die Frontkurve der Doppelscheiben 2, 3 angepasst werden. Dadurch vermeidet man eine Abflachung der Innenscheibe 3, die wiederum einen negativen Einfluss auf die Stabilität des Gesamtsystems ausüben könnte und negative Auswirkungen auf die Abbildungseigenschaften der optischen Linsen durch einen geänderten Durchblickwinkel hätte.

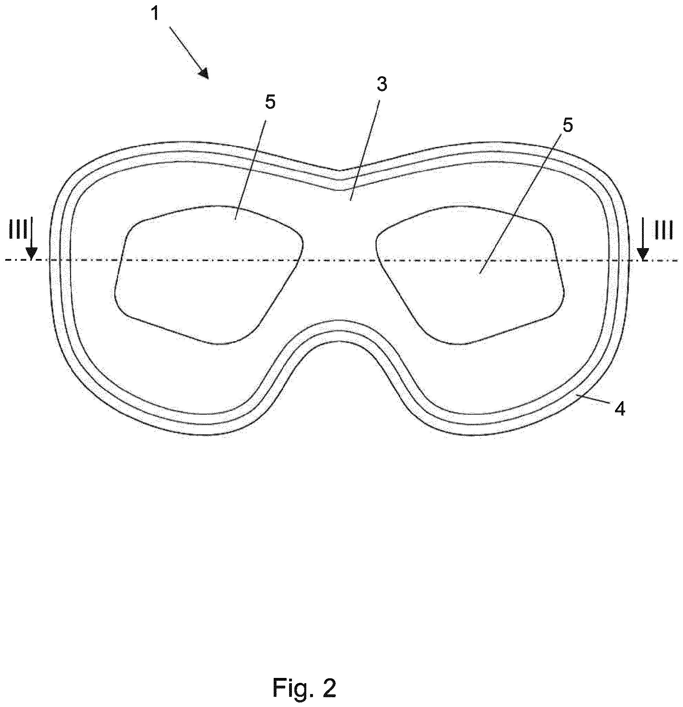

Fig. 2 zeigt eine weitere Ansicht der erfindungsgemäßen Sichtscheibe 1, wobei der Bereich des Schaumstoff-Dichtringes 4 besonders gekennzeichnet wurde.

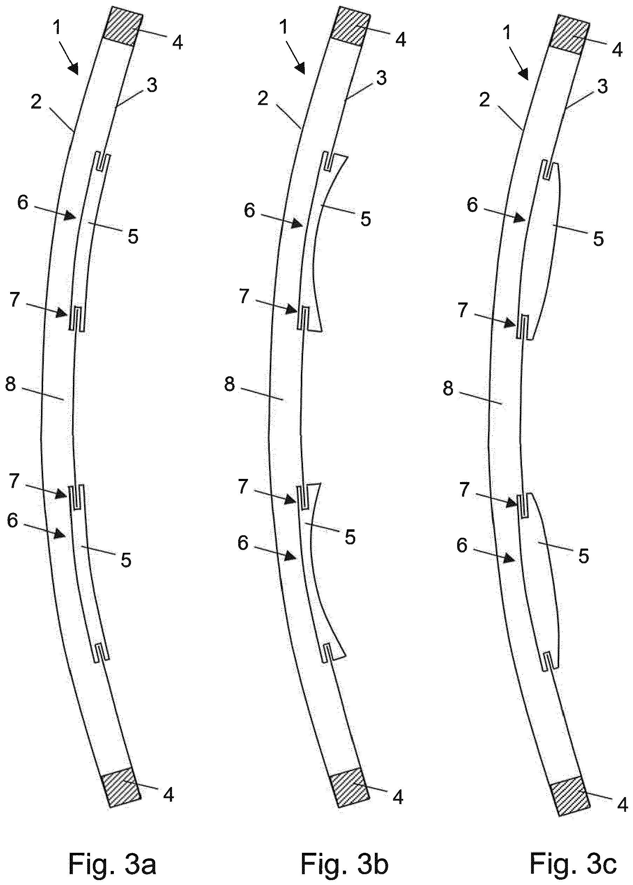

Figs. 3a - 3c zeigen drei Ausführungsformen der erfindungsgemäßen Sichtscheibe entlang des Schnittes III-III, der in Fig. 2 angedeutet ist. Die Ausführungsbeispiele unterscheiden sich durch die Ausführung der optischen Linsen 5, die in Fig. 3a konstante Dicke aufweisen, in Fig. 3b als plankonkave Linsen ausgeführt sind, und in Fig. 3c als plankonvexe Linsen ausgeführt sind. Allen Ausführungsformen ist gemein, dass die Frontkurve der optischen Linsen 5 im Wesentlichen parallel zur Frontkurve der Außenscheibe 2 verläuft, um Abbildungsfehler zu vermeiden.

Darüber hinaus sind in allen drei Ausführungsbeispielen die optischen Linsen 5 mit einer umlaufenden Nut 7 versehen, in welche der Rand der Ausnehmung 6 der Innenscheibe 3 eingreift, um die Linsen 5 in den Ausnehmungen 6 zu halten. Die Nut 7 ist im Wesentlichen im mittigen Bereich der Linsen 5 vorgesehen, um zu erreichen, dass das Eindringen der Linsen 5 in den Zwischenraum 8 zwischen Innenscheibe 3 und Außenscheibe 2 maximal etwa der Hälfte der Dicke des Zwischenraumes 8 entspricht.

Die Erfindung beschränkt sich nicht auf die dargestellten Ausführungsbeispiele sondern umfasst auch weitere Ausführungsbeispiele im Rahmen der Schutzansprüche. Die Erfindung umfasst insbesondere Brillen, vorzugsweise Skibrillen, Sportbrillen oder Schutzbrillen, oder Helmvisiere, sowie Schutzhelme für den polizeilichen oder militärischen Einsatz, sowie Feuerwehrschutzhelme, mit einer erfindungsgemäßen Sichtscheibe.

### Bezugszeichenliste

- 1: Sichtscheibe
- 2: Außenscheibe
- 3: Innenscheibe
- 4: Schaumstoff-Dichtring
- 5: Optische Linse
- 6: Ausnehmung
- 7: Nut
- 8: Zwischenraum

## Patentansprüche

1. Sichtscheibe (1) für eine Brille, vorzugsweise eine Sportbrille, Skibrille, Schutzbrille, oder für ein Helmvisier, umfassend zumindest eine Außenscheibe (2) und zumindest eine Innenscheibe (3), wobei die Außenscheibe in einem Abstand von der Innenscheibe angeordnet ist und mit dieser über ein randseitiges Dichtungsmittel verbunden ist, wobei in der Innenscheibe (3) zumindest eine optische Linse (5) zur Korrektur von Fehlsichtigkeiten integriert ist, **dadurch gekennzeichnet, dass** die Frontkurve der optischen Linse (5) im Zwischenraum (8) zwischen der Innenscheibe (3) und der Außenscheibe (2) im Wesentlichen parallel zur Frontkurve der Außenscheibe (2) verläuft, sodass Abbildungsfehler vermieden werden.

2. Sichtscheibe nach Anspruch 1, **dadurch gekennzeichnet, dass** zur Aufnahme der optischen Linse (5) die Innenscheibe (3) zumindest eine Ausnehmung (6) aufweist, die den Einbau der optischen Linse erlaubt.

3. Sichtscheibe nach Anspruch 2, **dadurch gekennzeichnet, dass** die optische Linse (5) in die Ausnehmung (6) geklebt ist oder in die Ausnehmung (6) klebbar ist.

4. Sichtscheibe nach Anspruch 2 oder 3, **dadurch gekennzeichnet, dass** die optische Linse (5) eine umlaufende Nut (7) zum Einschieben von zumindest Teilen des umlaufenden Randes der Ausnehmung (6) aufweist, sodass die optische Linse in die Ausnehmung (6) steckbar ist.

5. Sichtscheibe nach Anspruch 4, **dadurch gekennzeichnet, dass** die Nut (7) im Wesentlichen parallel zum Verlauf jenes umlaufenden Randes der optischen Linse (5) ausgeführt ist, der im Zwischenraum (8) zwischen Innenscheibe (3) und Außenscheibe (2) liegt.

6. Sichtscheibe nach einem der vorhergehenden Ansprüche, **dadurch gekennzeichnet, dass** die optische Linse (5) derart in der Innenscheibe (3) integriert ist, dass das Ausmaß des Vorsprungs der optischen Linse (5) in den Zwischenraum (8) zwischen Innenscheibe (3) und Außenscheibe (2) maximal der Hälfte der Dicke des Zwischenraumes (8) entspricht.

7. Sichtscheibe nach einem der vorhergehenden Ansprüche, **dadurch gekennzeichnet, dass** das Dichtungsmittel als ein längs der Randbereiche der Scheiben (2, 3) umlaufendes, elastisches Abstandhalte-Dichtungselement ausgeführt ist, welches mit der Außenscheibe (2) und der Innenscheibe (3) verbunden, vorzugsweise verklebt ist.

8. Sichtscheibe nach Anspruch 7, **dadurch gekennzeichnet, dass** das Abstandhalte-Dichtungselement als Schaumstoff-Dichtring (4) ausgeführt ist.

9. Sichtscheibe nach Anspruch 8, **dadurch gekennzeichnet, dass** der Schaumstoff-Dichtring (4) eine Breite von 3mm bis 5mm und eine Dicke von 3mm bis 5mm aufweist und mittels eines Doppelklebebandes mit den Scheiben (2, 3) verklebt ist.

10. Sichtscheibe nach einem der vorhergehenden Ansprüche, **dadurch gekennzeichnet, dass** die Außenscheibe (2) und/oder die Innenscheibe (3) und/oder die optische Linse (5) eine beschlaghemmende Beschichtung umfassen, wobei die die Innenscheibe (3) und/oder die optische Linse (5) vorzugsweise beidseitig mit einer beschlaghemmenden Beschichtung versehen ist.

11. Sichtscheibe nach einem der vorhergehenden Ansprüche, **dadurch gekennzeichnet, dass** die Außenscheibe (2) und/oder die Innenscheibe (3) mit einer lichtreflektierenden und/oder Infrarotstrahlung reflektierenden Beschichtung versehen ist.

12. Sichtscheibe nach einem der vorhergehenden Ansprüche, **dadurch gekennzeichnet, dass** in der Innenscheibe (3) zwei Ausnehmungen zur Integration optischer Linsen (5) vorgesehen sind.

13. Sichtscheibe nach einem der vorhergehenden Ansprüche, **dadurch gekennzeichnet, dass** die optischen Linsen (5) zur Korrektur von Kurzsichtigkeit, zur Korrektur von Fernsichtigkeit, als Bifokalgläser, oder als Gleitsichtgläser ausgeführt sind.

14. Brille, vorzugsweise Sportbrille, Skibrille oder Schutzbrille, oder Visier eines Helmes, insbesondere Visier für Feuerwehrhelme oder polizeiliche oder militärische Schutzhelme, umfassend eine Sichtscheibe nach einem der vorhergehenden Ansprüche.

15. Helm mit einem Helmvisier gemäß Anspruch 14.

## Claims

1. A viewing glass (1) for a pair of glasses, preferably a pair of sports glasses, ski goggles, safety glasses or for a helmet visor, comprising at least one outer glass (2) and at least one inner glass (3), the outer glass being arranged at a distance from the inner glass and being connected to the same via an edge-side sealing material, at least one optical lens (5) being integrated in the inner glass (3) for correcting defective eyesight, **characterized in that** the front curve of the optical lens (5) runs essentially parallel to the front curve of the outer lens (2) in the intermediate space (8) between the inner glass (3) and the outer glass (2), so that imaging errors are prevented.

2. The viewing glass according to Claim 1, **characterized in that** the inner glass (3) has at least one recess (6) for accommodating the optical lens (5), which recess enables the installation of the optical lens.

3. The viewing glass according to Claim 2, **characterized in that** the optical lens (5) is adhesively bonded into the recess (6) or can be adhesively bonded into the recess (6).

4. The viewing glass according to Claim 2 or 3, **characterized in that** the optical lens (5) has a circumferential groove (7) for inserting at least parts of the circumferential edge of the recess (6), so that the optical lens can be plugged into the recess (6).

5. The viewing glass according to Claim 4, **characterized in that** the groove (7) is designed to be substantially parallel to the course of the circumferential edge of the optical lens (5), which lies in the intermediate space (8) between inner glass (3) and outer glass (2).

6. The viewing glass according to one of the preceding claims, **characterized in that** the optical lens (5) is integrated in the inner glass (3) in such a manner that the extent of the projection of the optical lens (5) into the intermediate space (8) between inner glass (3) and outer glass (2) corresponds at most to half the thickness of the intermediate space (8).

7. The viewing glass according to one of the preceding claims, **characterized in that** the sealing material is designed as an elastic spacer sealing element running around along the edge regions of the glasses (2, 3), which is connected, preferably adhesively bonded, to the outer glass (2) and the inner glass (3).

8. The viewing glass according to Claim 7, **characterized in that** the spacer sealing element is designed as a foam sealing ring (4).

9. The viewing glass according to Claim 8, **characterized in that** the foam sealing ring (4) has a width from 3 mm to 5 mm and a thickness from 3 mm to 5 mm and is adhesively bonded to the glasses (2, 3) by means of double-sided adhesive tape.

10. The viewing glass according to one of the preceding claims, **characterized in that** the outer glass (2) and/or the inner glass (3) and/or the optical lens (5) comprise an anti-fog coating, wherein the inner glass (3) and/or the optical lens (5) is preferably provided with an anti-fog coating on both sides.

11. The viewing glass according to one of the preceding claims, **characterized in that** the outer glass (2) and/or the inner glass (3) is provided with a light-reflective and/or infrared-radiation-reflective coating.

12. The viewing glass according to one of the preceding claims, **characterized in that** two recesses are provided in the inner glass (3) for integrating optical lenses (5).

13. The viewing glass according to one of the preceding claims, **characterized in that** the optical lenses (5) are designed for correcting short-sightedness, for correcting long-sightedness, as bifocal lenses or as varifocal lenses.

14. A pair of glasses, preferably sports glasses, ski goggles or safety glasses, or a visor of a helmet, particularly visors for fire brigade helmets or police or military protective helmets, comprising a viewing glass according to one of the preceding claims.

15. A helmet having a helmet visor according to Claim 14.

## Revendications

1. Verre de vue (1) pour des lunettes, de préférence des lunettes de sport, des lunettes de ski, des lunettes de protection, ou pour un viseur de casque, comprenant au moins un verre extérieur (2) et au moins un verre intérieur (3), dans lequel le verre extérieur est disposé à une distance par rapport au verre intérieur et est relié avec celui-ci via un moyen d'étanchéité du côté du bord, dans lequel au moins une lentille optique (5) est intégrée dans le verre intérieur (3) pour la correction de déficiences visuelles, **caractérisé en ce que** la courbe frontale de la lentille optique (5) s'étend dans l'espace intermédiaire (8) entre le verre intérieur (3) et le verre extérieur (2) sensiblement parallèlement à la courbe frontale du verre extérieur (2) de sorte que des erreurs de représentation sont évitées.

2. Verre de vue selon la revendication 1, **caractérisé en ce que**, pour le logement de la lentille optique (5), le verre intérieur (3) présente au moins un évidement (6) qui permet l'encastrement de la lentille optique.

3. Verre de vue selon la revendication 2, **caractérisé en ce que** la lentille optique (5) est collée dans l'évidement (6) ou peut être collée dans l'évidement (6) .

4. Verre de vue selon la revendication 2 ou 3, **caractérisé en ce que** la lentille optique (5) présente une rainure périphérique (7) pour l'insertion de parties au moins du bord périphérique de l'évidement (6) de sorte que la lentille optique peut être placée dans l'évidement (6).

5. Verre de vue selon la revendication 4, **caractérisé en ce que** la rainure (7) est réalisée sensiblement parallèlement au tracé de ce bord périphérique de la lentille optique (5) qui se situe dans l'espace intermédiaire (8) entre le verre intérieur (3) et le verre extérieur (2).

6. Verre de vue selon l'une des revendications précédentes, **caractérisé en ce que** la lentille optique (5) est intégrée dans le verre intérieur (3) de telle sorte que le degré de projection de la lentille optique (5) dans l'espace intermédiaire (8) entre le verre intérieur (3) et le verre extérieur (2) correspond au maximum à la moitié de l'épaisseur de l'espace intermédiaire (8).

7. Verre de vue selon l'une des revendications précédentes, **caractérisé en ce que** le moyen d'étanchéité est réalisé en tant qu'élément d'étanchéité d'espacement élastique faisant le tour le long des zones de bord des verres (2, 3) lequel est relié, de préférence collé, au verre extérieur (2) et au verre intérieur (3).

8. Verre de vue selon la revendication 7, **caractérisé en ce que** l'élément d'étanchéité d'espacement est réalisé en tant que bague d'étanchéité en matière alvéolaire (4).

9. Verre de vue selon la revendication 8, **caractérisé en ce que** la bague d'étanchéité en matière alvéolaire (4) présente une largeur de 3 mm à 5 mm et une épaisseur de 3 mm à 5 mm et est collée aux verres (2, 3) au moyen d'une bande adhésive double face.

10. Verre de vue selon l'une des revendications précédentes, **caractérisé en ce que** le verre extérieur (2) et/ou le verre intérieur (3) et/ou la lentille optique (5) comprennent un revêtement antibuée, dans lequel le verre intérieur (3) et/ou la lentille optique (5) sont de préférence dotés d'un revêtement antibuée des deux côtés.

11. Verre de vue selon l'une des revendications précédentes, **caractérisé en ce que** le verre extérieur (2) et/ou le verre intérieur (3) sont dotés d'un revêtement de réflexion de lumière et/ou de réflexion du rayonnement infrarouge.

12. Verre de vue selon l'une des revendications précédentes, **caractérisé en ce que** l'on prévoit deux évidements dans le verre intérieur (3) pour l'intégration de lentilles optiques (5).

13. Verre de vue selon l'une des revendications précédentes, **caractérisé en ce que** les lentilles optiques (5) sont réalisées pour la correction de la myopie, la correction de la vision de loin, en tant que verres bifocaux, ou en tant que verres progressifs.

14. Lunettes, de préférence lunettes de sport, lunettes de ski ou lunettes de protection, ou viseur d'un casque, en particulier viseur pour casques de pompiers ou casques de protection de police ou militaires, comprenant un verre de vue selon l'une des revendications précédentes.

15. Casque avec un viseur de casque selon la revendication 14.
